# EUROPEAN PATENT SPECIFICATION

(11) **EP 2 991 585 B1**
(45) Date of publication and mention of the grant of the patent: **16.08.2023**
(21) Application number: 14729789.9
(22) Date of filing: 02.05.2014
(51) Int. Cl.: A61F 2/24

(54) **MEDICAL DEVICES FOR IMPLANTING IN A VALVE**
MEDIZINISCHE VORRICHTUNGEN ZUR IMPLANTATION IN EINER KLAPPE
DISPOSITIFS MÉDICAUX DESTINÉS À ÊTRE IMPLANTÉS DANS UNE VALVE

(30) Priority: 03.05.2013 US 201361819486 P; 23.01.2014 US 201461930851 P
(43) Date of publication of application: 09.03.2016
(73) Proprietor: Medtronic Inc., Minneapolis, MN 55432 (US)
(72) Inventor: WESTON, Matthew, Santa Rosa, CA 95403 (US); KIM, Stella, Santa Rosa, CA 95403 (US); MAJKRZAK, Carolyn C., Santa Rosa, CA 95403 (US); GLOSS, Michael, Santa Rosa, CA 95403 (US); GROEN, Timothy, Santa Rosa, CA 95403 (US); NADIAN, Behrooz, Santa Rosa, CA 95403 (US); RUST, Matthew, Santa Rosa, CA 95403 (US); RYAN, Timothy, Santa Rosa, CA 95403 (US)
(74) Representative: Zimmermann & Partner Patentanwälte mbB
(86) International application number: PCT/US2014/036686
(87) International publication number: WO 2014/179761

(56) References cited:
- WO-A1-01/47438
- US-A1- 2002 087 186
- US-A1- 2003 070 676
- US-A1- 2006 206 202
- US-A1- 2007 043 435
- US-A1- 2009 192 591
- US-A1- 2009 292 262

## Description

### FIELD

The present disclosure relates to, among other things, heart valves, and associated apparatuses and methods of use, manufacture and treatment.
WO 01/47438 A1 describes enhanced visualization of medical implants. US 2006/0206202 A1 describes an apparatus for treatment of cardiac valves and method of its manufacture. US 2009/0192591 A1 describes markers for prosthetic heart valves. US 2009/0292262 A1 describes a valve loader method, system, and apparatus. US 2003/0070676 A1 describes conduits having distal cage structure for maintaining collateral channels in tissue and related methods. US 2007/0043435 A1 describes a non-cylindrical prosthetic valve system for transluminal delivery. US 2002/0087186 A1 describes an expansion-assisting delivery system for self-expanding stent.

### BACKGROUND

The transport of vital fluids in the human body is largely regulated by valves. Physiological valves are designed to prevent the backflow of bodily fluids, such as blood, lymph, urine, bile, etc., thereby keeping the body's fluid dynamics unidirectional for proper homeostasis. For example, venous valves maintain the upward flow of blood, particularly from the lower extremities, back toward the heart, while lymphatic valves prevent the backflow of lymph within the lymph vessels, particularly those of the limbs.

A human heart includes four cardiac valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart.

Because of their common function, valves share certain anatomical features despite variations in relative size. Cardiac valves are among the largest valves in the body with diameters that may exceed 30 mm, while valves of smaller veins may have diameters no larger than a fraction of a millimeter. Regardless of their size, however, some physiological valves are situated in specialized anatomical structures known as sinuses. Valve sinuses can be described as dilations or bulges in the vessel wall that houses the valve. The geometry of the sinus has a function in the operation and fluid dynamics of the valve. One function is to guide fluid flow so as to create eddy currents that prevent the valve leaflets from adhering to the wall of the vessel at the peak of flow velocity, such as during systole. Another function of the sinus geometry is to generate currents that facilitate the precise closing of the leaflets at the beginning of backflow pressure. The sinus geometry is also important in reducing the stress exerted by differential fluid flow pressure on the valve leaflets or cusps as they open and close.

Sinuses of the pulmonary trunk comprise the space at the origin of the pulmonary trunk between the dilated wall of the vessel and each cusp of the pulmonic valve. Aortic sinuses or Valsalva sinuses comprise the space between the superior aspect of each cusp of the aortic valve and the dilated portion of the wall of the ascending aorta, immediately above each cusp. Thus, for example, eddy currents occurring within sinuses of Valsalva in the natural aortic root have been shown to be important in creating smooth, gradual and gentle closure of the aortic valve at the end of systole. Blood is permitted to travel along the curved contour of the sinus and onto the valve leaflets to effect their closure, thereby reducing the pressure that would otherwise be exerted by direct fluid flow onto the valve leaflets. The sinuses of Valsalva also contain the coronary ostia, which are outflow openings of the arteries that feed the heart muscle. When valve sinuses contain such outflow openings, they serve the additional purpose of providing blood flow to such vessels throughout the cardiac cycle.

When valves exhibit abnormal anatomy and function as a result of valve disease or injury, the unidirectional flow of the physiological fluid they are designed to regulate is disrupted, resulting in increased hydrostatic pressure. For example, venous valvular dysfunction leads to blood flowing back and pooling in the lower legs, resulting in pain, swelling and edema, changes in skin color, and skin ulcerations that can be extremely difficult to treat. Lymphatic valve insufficiency can result in lymphedema with tissue fibrosis and gross distention of the affected body part. Cardiac valvular disease may lead to pulmonary hypertension and edema, atrial fibrillation, and right heart failure in the case of mitral and tricuspid valve stenosis; or pulmonary congestion, left ventricular contractile impairment and congestive heart failure in the case of mitral regurgitation and aortic stenosis. Regardless of their etiology, all valvular diseases result in either stenosis, in which the valve does not open properly, impeding fluid flow across it and causing a rise in fluid pressure, or insufficiency/regurgitation, in which the valve does not close properly and the fluid leaks back across the valve, creating backflow. Some valves are afflicted with both stenosis and insufficiency, in which case the valve neither opens fully nor closes completely.

Because of the potential severity of the clinical consequences of valve disease, numerous surgical techniques have been developed to repair a diseased or damaged heart valve. For example, these surgical techniques may include annuloplasty (contracting the valve annulus), quadrangular resection (narrowing the valve leaflets), commissurotomy (cutting the valve commissures to separate the valve leaflets), or decalcification of valve and annulus tissue. Alternatively, the diseased heart valve may be replaced by a prosthetic valve. Where replacement of a heart valve is indicated, the dysfunctional valve is typically removed and replaced with either a mechanical or tissue valve.

In the past, one common procedure has been an open-heart type procedure. However, open-heart valve repair or replacement surgery is a long and tedious procedure and involves a gross thoracotomy, usually in the form of a median sternotomy. In this procedure, a saw or other cutting instrument is used to cut the sternum longitudinally and the two opposing halves of the anterior or ventral portion of the rib cage are spread apart. A large opening into the thoracic cavity is thus created, through which the surgeon may directly visualize and operate upon the heart and other thoracic contents. The patient must typically be placed on cardiopulmonary bypass for the duration of the surgery.

Minimally invasive valve replacement procedures have emerged as an alternative to open-chest surgery. Minimally invasive medical procedures may be considered as procedures that are carried out by entering the body through the skin or through a body cavity or anatomical opening, while minimizing damage to these structures. Two types of minimally invasive valve procedures that have emerged are percutaneous valve procedures and trans-apical valve procedures. Percutaneous valve procedures pertain to making small incisions in the skin to allow direct access to peripheral vessels or body channels to insert catheters. Trans-apical valve procedures pertain to making a small incision in or near the apex of a heart to allow valve access.

Traditionally, surgical heart valves have been implanted with a multitude of sutures, so placing the valve at the correct depth was readily accomplished by tactile means. For sutureless valves, such tactile feedback does not exist. Accordingly, alternatives for ensuring proper depth of an implant of sutureless valves would be desirable.

Additionally, ensuring proper orientation of a valve apparatus is fairly routine heart valves anchored via sutures. However, with heart valves that are expandable and initially implanted in a collapsed configuration, ensuring proper orientation can be more challenging.

### SUMMARY

The invention relates to a device configured to be implanted in a valve of a subject as defined in attached claim 1.

Described herein are, among other things, prosthetic heart valves having visible markings configured to be aligned with anatomical structures of a native valve, such as an annulus or a commissure. The markings facilitate accurate implantation of the prosthetic valves at a proper depth or in a proper orientation.

In some embodiments, a device configured to be implanted in a valve of a subject is described. The valve includes an annulus. According to the invention, the device comprises a frame having an annular portion configured to be aligned with the annulus of the native valve and a visible circumferential marking positioned around the annular portion of the frame.

The frame, in some embodiments, is expandable from a collapsed configuration to an expanded configuration. In the expanded configuration, the annular portion of the frame is configured to engage the annulus of the native valve. The frame may be configured to be at least partially collapsed from the expanded configuration to an at least partially collapsed configuration such that the frame can be repositioned during an implant procedure if the visible circumferential marking is not aligned with the annulus of the native valve.

The frame, in some embodiments, is expandable from a collapsed configuration to a partially expanded configuration. When the frame is in the partially expanded configuration, the device is configured such that circumferential marking is visible when aligned with the annulus of the native valve.

In some embodiments, the frame has a flange positioned superior to the annular portion when implanted, wherein the flange is compressible and expandable. The flange may be a part of a concave-shaped portion configured to anchor the device around the annulus of the native valve.

According to the invention, the device includes a skirt disposed over at least a portion of the frame. The visible circumferential marking may be disposed on the skirt.

According to the invention, the device further includes a second visible circumferential marking positioned around the frame at a location that, when implanted, is superior to the first visible circumferential marking. The second circumferential marking indicates a position to which a sheath of a delivery system is to be withdrawn during a part of an implant procedure in which the device is being positioned within the native valve.

In some embodiments, a device includes a frame that has a longitudinal portion configured to be aligned with a commissure of the native valve. The device further comprises one or more visible commissural alignment markings positioned to indicate at least a portion of the longitudinal portion of the frame. The device, according to the invention, includes a skirt disposed about at least a portion of the frame. The one or more visible commissural alignment markings may be disposed on the skirt.

In various embodiments, the device is a prosthetic heart valve. In some embodiments the prosthetic heart valve is a sutureless prosthetic heart valve.

Further disclosed but not claimed is a method for implanting a device in a valve of a subject. A portion of the device is configured to be aligned with an annulus of the valve. The device has a visible circumferential marking configured to be aligned with the valve annulus. In some embodiments, the method may include inserting at least a portion of the device in a valve sinus, and aligning a visible circumferential marking with the valve annulus. In some embodiments, the device is compressible and expandable, and inserting the device in a native valve comprises inserting the device in an at least partially compressed configuration. The method in such embodiments may further comprise expanding the device, or allowing the device to expand, when the visible circumferential marking is aligned with the native valve annulus. In some embodiments, the device comprises a second visible circumferential marking that when implanted is superior to the first visible circumferential marking. In such embodiments, the method may further include retracting a retention sheath about the device until the second circumferential marking is visible to allow the device to partially expand prior to aligning the first circumferential marking with the native valve annulus. The method may further include completely retracting the sheath about the device when the first visible circumferential marking is aligned with the annulus to allow the device to expand and engage the native valve annulus.

Advantages of one or more of the various embodiments presented herein over prior devices for implanting in a valve of a patient, such as prosthetic heart valves, and associated methods will be readily apparent to those of skill in the art based on the following detailed description when read in conjunction with the accompanying drawings.

### BRIEF DESCRIPTION OF THE DRAWINGS

**FIG. 1A** is a schematic drawing of an exemplary valve in an open position during peak flow.
**FIG. 1B** is a schematic drawing of the valve of **FIG. 1A** in a closed position to prevent backflow of the fluid across the valve.
**FIG. 2A** is a schematic drawing of a top view illustrating the anatomy of a typical aortic valve.
**FIG. 2B** is a schematic drawing of a cross-sectional view of the aortic valve of **FIG. 2A****.**
**FIG. 2C** is a schematic perspective view of the aortic valve of **FIG. 2A** showing the inflow end, outflow end, and commissural posts in phantom lines.
**FIG. 3** is a schematic representation of the geometry and relative dimensions of the valve sinus region.
**FIG. 4** is a schematic perspective view of a valve replacement system, which includes a replacement valve, a valve support structure (or "frame"), and a valve cuff.
**FIG. 5** is a schematic perspective view of the replacement valve of **FIG. 4****.**
**FIG. 6** is a schematic side view of the valve support structure of **FIG. 4** disposed inside a vessel.
**FIG. 7** is a schematic side view of the replacement valve system of **FIG. 4****.**
**FIG. 8** is a schematic view of the replacement valve system of **FIGS. 4** and 7 positioned within an aorta.
**FIG. 9A** is a schematic drawing of an embodiment of a support frame cut along line A-A and laid flat.
**FIG. 9B** is a schematic drawing of a cross-sectional view illustrating the concave landing zone of the frame of **FIG. 9A****.**
**FIG. 10** is a schematic drawing of an embodiment of a valve replacement system positioned in an aorta.
**FIGS. 11-15** are schematic drawings showing embodiments of prototype valve replacement systems including some embodiments of exemplary markings, wherein Figs. 15A and 15B show embodiments of the invention.

The schematic drawings in are not necessarily to scale. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar.

### DETAILED DESCRIPTION

The present disclosure relates to, among other things, devices for implantation in a valve, such as heart valves, and methods, systems, and devices associated therewith. The devices described herein may be particularly useful where tactile feedback of valve positioning is not possible or impracticable, such as when implanting sutureless prosthetic heart valve devices.

In various embodiments, the implantable devices described herein have visible markings configured to be aligned with anatomical structures of a valve, such as an annulus or a commissure. The markings facilitate accurate implantation of the prosthetic valves at a proper depth or in a proper orientation.

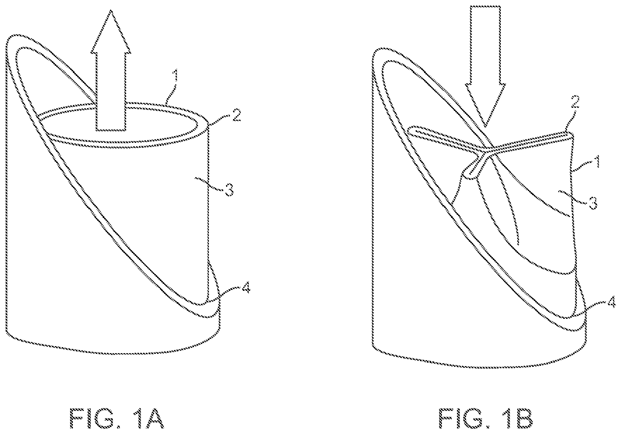

Prior to describing devices with such markings, a general description of heart valve device components and heart valve anatomy is provided with regard to **FIGS. 1-8****.**

**FIGS. 1A** and **1B** generally illustrate one exemplary embodiment of a heart valve **1.** As illustrated in **FIG. 1****,** valve **1** includes a distal outflow end **2,** a plurality of leaflets **3,** and a proximal inflow end **4.** A typical valve functions similar to a collapsible tube in that it opens widely during systole or in response to muscular contraction to enable unobstructed forward flow across the valvular orifice, as illustrated in **FIG. 1A****.** In contrast, as forward flow decelerates at the end of systole or contraction, the walls of the tube are forced centrally between the sites of attachment to the vessel wall and the valve closes completely as illustrated in **FIG. 1B****.**

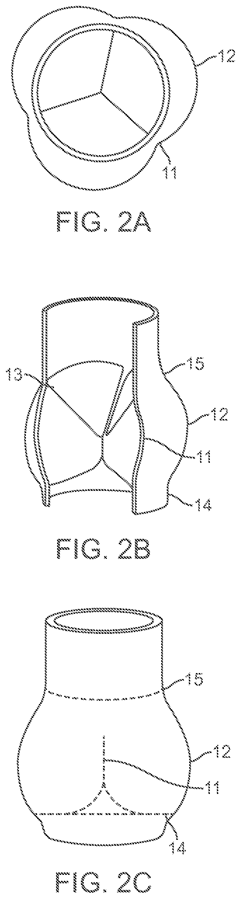

**FIGS. 2A, 2B,** and **2C** illustrate the anatomy of a typical aortic valve. In particular, **FIG. 2A** shows a top view of a closed valve with three valve sinuses, **FIG. 2B** shows a perspective sectional view of the closed valve, and **FIG. 2C** shows a view from outside the vessel wall.

One consideration in the design of valve replacement systems and devices is the architecture of the valve to be replaced. For example, mitral and tricuspid heart valves do not have valve sinuses whereas aortic and pulmonic heart valves have valve sinuses. Valve sinuses **12** are dilations of the vessel wall that surround the natural valve leaflets. Typically in the aortic valve, each natural valve leaflet has a separate sinus bulge **12** or cavity that allows for maximal opening of the leaflet at peak flow without permitting contact between the leaflet and the vessel wall. As illustrated in **FIGS. 2A, 2B,** and **2C****,** the extent of the sinus **12** is generally defined by the commissures **11,** vessel wall **13,** inflow end **14,** and outflow end **15.** The proximal intersection between the sinus cavities define the commissures **11.**

**FIGS. 2B** and **2C** also show the narrowing diameter of the sinuses at both inflow end **14** and outflow end **15,** thus forming the inflow and outflow annuli of the sinus region. Thus, the valve sinuses form a natural compartment to support the operation of the valve by preventing contact between the leaflets and the vessel wall, which, in turn, may lead to adherence of the leaflets or result in detrimental wear and tear of the leaflets. The valve sinuses are also designed to share the stress conditions imposed on the valve leaflets during closure when fluid pressure on the closed leaflets is greatest. The valve sinuses further create favorable fluid dynamics through currents that soften an otherwise abrupt closure of the leaflets under conditions of high backflow pressure. Lastly, the sinuses ensure constant flow to any vessels located within the sinus cavities.

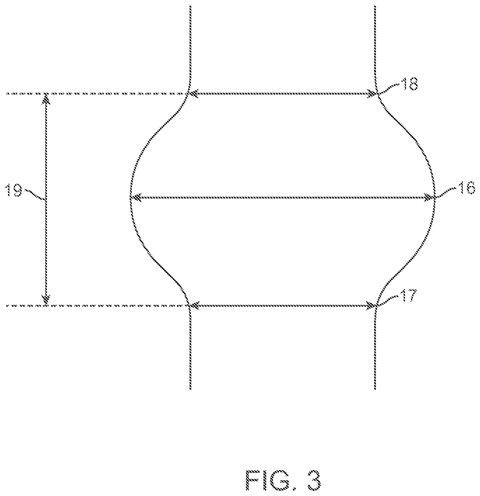

**FIG. 3** is a schematic representation of the geometry and relative dimensions of the valve sinus region. As shown in **FIG. 3****,** the valve sinus region is characterized by certain relative dimensions which remain substantially constant regardless of the actual size of the sinuses. Generally, the diameter of the sinus is at its largest at the center of the sinus cavities **16,** while there is pronounced narrowing of the sinus region at both the inflow annulus **17** near the inflow end **14** and the outflow annulus **18** near the outflow end **15.** Furthermore, the height of the sinus **19** (i.e. the distance between inflow annulus **17** and outflow annulus **18**) remains substantially proportional to its overall dimensions. It is thus apparent that the sinus region forms an anatomical compartment with certain constant features that are uniquely adapted to house a valve. The systems and devices disclosed herein may be designed to utilize these anatomical features of the native sinus region for replacement valve function and positioning.

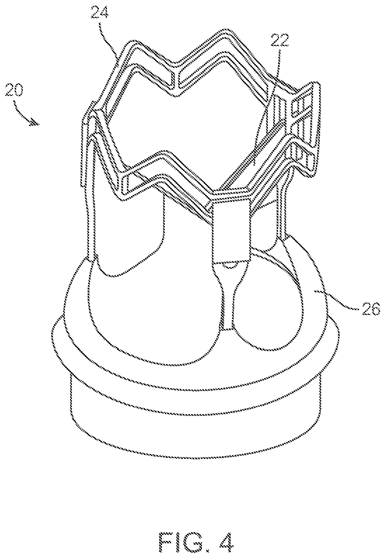

**FIG. 4** is a perspective view of a valve replacement system **20** described in more detail in US Published Patent Application No. 2010/0168844, which contains general features of the valves described in more detail below. Such valves, as well as the valve depicted in **FIG. 4**, include replacement valve **22**, valve support structure or frame **24**, and valve cuff **26.** Replacement valve **22** may be attached to frame **24** such that replacement valve **22** resides within the support structure. Valve support structure **24** may be, for example, an expandable and collapsible stent-like frame structure adapted to be delivered to an implantation site such as a native heart valve. Frame **24** may be either self-expanding or non-self-expanding, and may be delivered to the target site via any suitable delivery means as will be appreciated by one skilled in the art. Valve cuff **26** is attachable to the inflow end of replacement valve **22**, and may be structured to reduce paravalvular leakage around the valve, as well as to reduce migration and increase stability of replacement valve **22** after implantation at the implantation site.

Replacement valve **22** illustrated in **FIG. 4** is a tri-leaflet valve. For purposes of example and not limitation, the following discussion will reference only valve **22**, it being understood that any stented or stentless replacement valve is contemplated. Similarly, although valve frame **24** is shown as structured to receive a tri-leaflet valve, those skilled in the art will appreciate that replacement valves having a number of leaflets other than three will correspondingly require a different valve support structure.

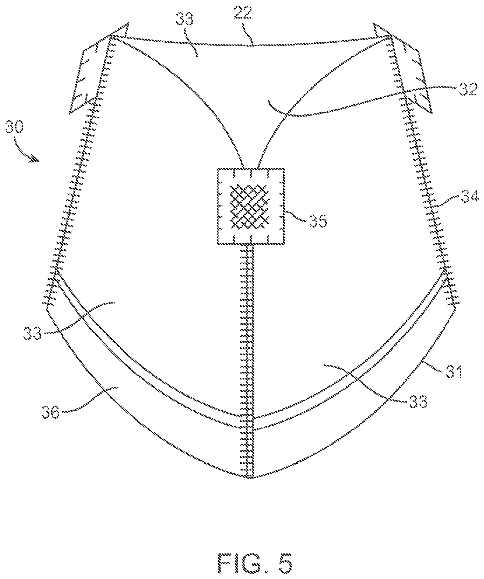

**FIG. 5** is a perspective view of replacement valve **22**, which represents one exemplary embodiment of a tri-leaflet replacement valve useable with valve replacement systems **20** described herein. Replacement valve **22** includes valve body **30** having proximal inflow end **31** and a distal outflow end **32.** Valve body **30** includes a plurality of valve tissue leaflets **33** joined by seams **34**, wherein each seam **34** is formed by a junction of two leaflets **33.** A commissural tab region **35** extends from each seam **34** at the distal end of valve body **30.** Inflow end **31** of valve body **30** includes a peripheral edge that may be scalloped or straight. In addition, inflow end **31** of valve body **30** may further comprise reinforcement structure **36** that may be stitched or otherwise attached thereto.

The valve replacement systems and devices described herein are not limited, however, to the specific valve illustrated in **FIG. 5****.** For example, although the proximal inflow end **31** of valve body **30** is shown in **FIG. 5** with a scalloped peripheral edge, other shapes and configurations are contemplated and within the intended scope of the present disclosure.

Valve leaflets **33** may be constructed of any suitable material, including but not limited to polymeric materials, metallic materials, or tissue-engineered materials. For example, bovine, porcine, equine, ovine, or other suitable animal tissues may be used to construct valve leaflets. In some embodiments, valve leaflets may be constructed of or formed from material obtained from, for example, heart valves, aortic roots, aortic walls, aortic leaflets, pericardial tissue, blood vessels, intestinal submucosal tissue, umbilical tissue and the like from humans or animals. In some embodiments, valve leaflets may be constructed of expanded polytetrafluoroethylene (ePTFE), equine pericardium, bovine pericardium, or native porcine valve leaflets similar to currently available bioprosthetic aortic valves. Other materials may prove suitable as will be appreciated by one skilled in the art.

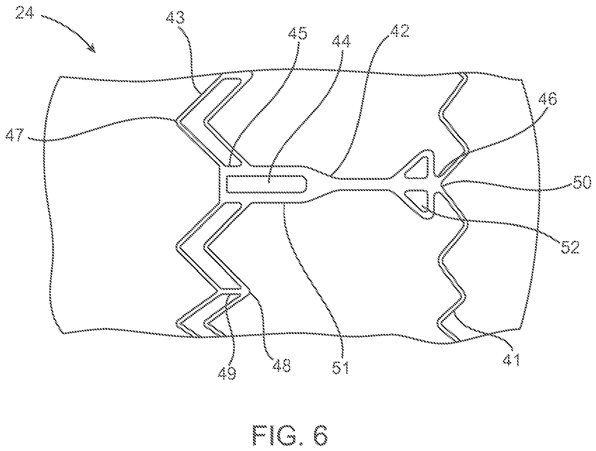

**FIG. 6** is a side view of valve support structure **24**, which represents one exemplary embodiment of a typical support structure useable with valve replacement system **20** in accordance with the teaching presented herein. In general, valve support structure **24** is designed as a collapsible and expandable anchoring structure that may be adapted to support valve **22** distally along commissural tab region **35** and proximally along the proximal inflow end **31.** As shown in **FIG. 6**, valve **22** and valve cuff **26** have been detached from valve frame **24** so as to focus on the structure and features of the support structure.

In some embodiments, valve frame **24** has a generally tubular configuration within which replacement valve **22** may be secured, and includes inflow rim **41**, support posts **42** and outflow rim **43.** Replacement valve **22** may be secured at the proximal inflow end **31** by attachment to inflow rim **41** of support structure **24** and at the distal outflow end **32** via commissural tabs **35** that are threaded through axially extending slots **44**, which are formed in support posts **42** that extend longitudinally from inflow rim **41** to outflow rim **43** of valve support structure **24.** Thus, distal ends **45** of support posts **42** contact outflow rim **43** of valve support structure **24**, whereas proximal ends **46** of support posts **42** contact inflow rim **41** of valve support structure **24.**

In the embodiment shown in **FIG. 6**, outflow rim **43** of support structure **24** is depicted as comprising a plurality of rings that extend between support posts **42** generally at or above the axially extending slots **44** that reside therein. The plurality of rings of outflow rim **43** are configured in an undulating or zigzag pattern forming peaks **47** and valleys **48**, wherein the individual rings remain substantially parallel to one another. The plurality of rings of outflow rim **43** may include a vertical connector element **49** positioned at the center of valleys **48** formed by the undulating or zigzag pattern. Vertical connector element **49** is designed to stabilize frame **24** and to prevent distortion of the valve during compression and expansion of the frame. Vertical element **49** extends longitudinally in the axial direction of the cylindrical valve support structure **24.**

In the embodiment of valve support structure **24** illustrated in **FIG. 6**, outflow rim **43** is formed with two rings, while inflow rim **41** is formed with a single ring that extends between support posts **42.** However, the number of rings is not important, and numerous other configurations are contemplated. For example, in the embodiments of valve support structure **24** illustrated in **FIGS. 4****,** **7****, and** **8**, inflow rim **41** is formed with two rings that extend between support posts **42.**

Both inflow rim **41** and outflow rim **43** of valve support structure **24** are formed with an undulating or zigzag configuration. In various embodiments of valve support structures, inflow rim **41** may have a shorter or longer wavelength (i.e., circumferential dimension from peak to peak) or a lesser or greater wave height (i.e., axial dimension from peak to peak) than outflow rim **43.** The wavelengths and wave heights of inflow rim **41** and outflow rim **43** may be selected to ensure uniform compression and expansion of valve support structure **24** without substantial distortion. The wavelength of inflow rim **41** is further selected to support the geometry of the inflow end of the valve attached thereto, such as the scalloped inflow end **31** of replacement valve **22** shown in **FIG. 5****.** Notably, as shown in **FIG. 6**, the undulating or zigzag pattern that forms inflow rim **41** of valve support structure **24** is configured such that proximal ends **46** of vertical support posts **42** are connected to peaks **50** of inflow rim **41.** Similarly, the undulating or zigzag pattern that forms outflow rim **43** of support structure **24** is configured such that distal ends **45** of support posts **42** are connected to valleys **48** of outflow rim **43.** Locating distal ends **45** of support posts **42** at valleys **48** of outflow rim **43** may prevent the longitudinal extension of outflow rim **43** in the direction of replacement valve **22** secured within the lumen of valve support structure **24** upon compression of the replacement valve assembly **20.** As a result, most, if not all, contact between replacement valve **22** and valve support structure **24** is eliminated. Likewise, locating proximal ends **46** of support posts **42** at peaks **50** of inflow rim **41** may prevent longitudinal extension of inflow rim **41** in the direction of the valve tissue. Thus, compression of replacement valve **22** and valve support structure **24** does not lead to distortion of or injury to the valve.

**FIG. 6** further shows that support posts **42** are configured generally in the shape of a paddle with axial slot **44** extending internally within blade **51** of the paddle. Blade **51** of the paddle is oriented toward outflow rim **43** of support structure **24** and connects to outflow rim **43** at a valley **48** of the undulating or zigzag pattern of outflow rim **43.** An important function of support posts **42** is the stabilization of valve **22** in general, and in particular the prevention of any longitudinal extension at points of valve attachment to preclude valve stretching or distortion upon compression of replacement valve system **20.** Blades **51** of the paddle-shaped support posts **42** may be designed to accommodate commissural tabs **35** of valve **22.**

Support posts **42** further comprise triangular shaped elements **52** extending on each side of proximal end **46** of the support post. Triangular shaped elements **52** may be designed to serve as attachments sites for valve cuff **26** and may be designed in different shapes without losing their function. Thus, the particular design of elements **52** shown in **FIG. 6** is not critical to the attachment of valve cuff **26**, and numerous other designs and shapes are contemplated and within the intended scope of the present disclosure.

The number of support posts **42** generally ranges from two to four, and generally depends on the number of commissures and leaflets present in the replacement valve 22. Thus, valve support structure **24** may comprise three support posts for a tri-leaflet replacement valve **22.** Support posts **32** of valve frame **24** may be structured to generally coincide with the natural commissures of the native valve being replaced.

Valve frame **24** may be formed from any suitable material including, but not limited to, stainless steel or nitinol. The particular material selected for valve support structure **24** may be determined based upon whether the support structure is self-expanding or non-self-expanding. For example, preferable materials for self-expanding support structures include shape memory materials, such as nitinol.

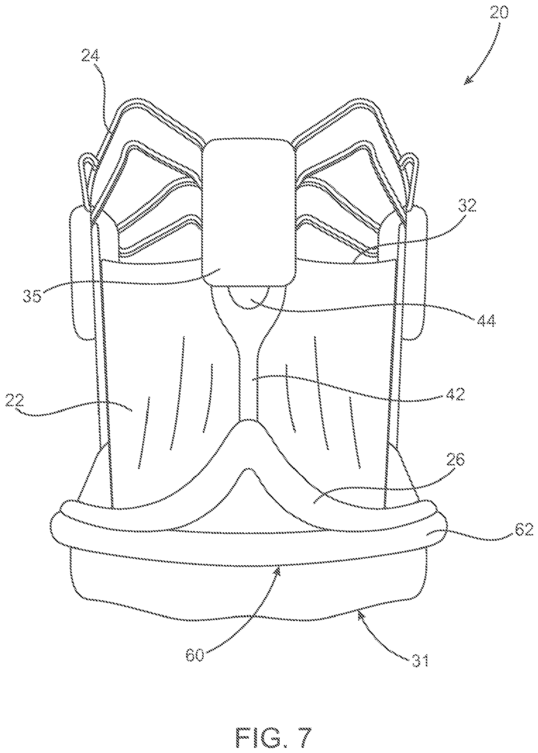

**FIG. 7** is a side view illustrating replacement valve device **20** of **FIG. 4****,** which once again includes replacement valve **22,** valve support frame **24,** and valve cuff **26.** As shown in the embodiment depicted in **FIG. 7****,** valve **22** is secured at the proximal inflow end **31** by attachment to inflow rim **41** of valve frame **24** and at the distal outflow end **32** via commissural tabs **35** that are threaded through axially extending slots **44** formed in support posts **42.** Notably, as can be seen in the embodiment shown in **FIG. 7****,** outflow rim **43** of frame **24** is structured to be longitudinally displaced from the distal outflow end **32** of valve leaflets **33** that reside within the lumen of the tubular valve frame **24.** Thus, contact between valve leaflets **33** and frame **24** is avoided.

The positioning of replacement valve **22** internally to frame **24** with only commissural mounting tabs **35** of replacement valve **22** contacting support posts **42** at the distal outflow end **32** of the valve, while the proximal inflow end **31** of the valve is separated from inflow rim **41** of valve support structure **24** by valve cuff **26,** ensures that no part of replacement valve **22** is contacted by frame **24** during operation of valve **22,** thereby eliminating wear on valve **22** that may be otherwise result from contact with mechanical elements.

As shown in **FIG. 7****,** valve cuff **26** generally includes skirt **60** and flange **62.** As illustrated in **FIG. 7****,** skirt **60** may be structured to cover the outer surface of valve support structure 24, such as along the proximal inflow end **31.** In particular, skirt **60** of valve cuff **26** wraps around the entire circumference of replacement valve **22** and frame **24** near the proximal inflow end **31** and inflow rim **41,** respectively. Furthermore, as shown in **FIG. 7****,** skirt **60** may have a generally scalloped configuration so as to substantially align with the scallops found in or around the native valve implantation site and with the scalloped configuration of replacement valve **22.** However, one skilled in the art will appreciate that valve cuffs with nonscalloped skirts are also contemplated and within the intended scope of the present disclosure.

Skirt **60** of valve cuff **26** is designed to provide numerous benefits when used in conjunction with a replacement valve such as replacement valve **22.** First, skirt **60** functions to protect the proximal inflow end **31** of replacement valve **22** from irregularities of a valve annulus such that, for example, calcification remnants or valve remnants left behind after a native valve removal procedure do not come into contact with any portion of replacement valve **22.** If otherwise allowed to contact replacement valve **22,** these remnants impose a risk of damage to the valve. Second, when positioned adjacent a native valve annulus, skirt **60** provides another source of valve sealing, and also assists valve cuff **26** to conform to irregularities of the native valve annulus. Third, once valve cuff **26** is positioned adjacent a native valve annulus, skirt **60** allows tissue ingrowth into the valve cuff. Such tissue ingrowth not only improves the seal provided by valve cuff **26,** but also helps to anchor the valve cuff to the native valve annulus and minimize migration of replacement valve system **20** after implantation. Skirt **60** of valve **cuff 26** may provide addition benefits other than those previously discussed as will be appreciated by those skilled in the art.

As illustrated in **FIG. 7****,** flange **62** of valve cuff **26** is coupled to skirt **60** and is structured to protrude from replacement valve system **20** around the entire circumference of the valve. Once replacement valve system **20** is delivered to an implantation site and deployed, valve support structure **24** exerts a radial force within valve cuff **26** which pushes flange **62** against native tissue at the implantation site, thereby creating a seal to prevent paravalvular leakage and migration of replacement valve system **20** within the aorta. For example, in embodiments where valve support structure **24** is formed from a memory shaped metal, the radial force may result from the support structure "springing" back to expanded form after deployment at the implantation site.

Flange **62** of valve cuff **26** is structured for forming a seal between the proximal inflow end **31** of replacement valve **22** and the annulus of the native valve site. In some embodiments, if one or more native valve structures are removed from a patient's body prior to implantation of replacement valve system **20,** irregularities may exist around the annulus of the native valve site. These irregularities may be the result of, for example, natural calcifications or valve remnants left over from extraction of the native valve. Irregularities around the annulus can be problematic because they can contribute to paravalvular leakage.

In the past when irregularities were present, it was difficult to maintain a tight seal between the native valve annulus and the replacement valve. However, flange **62** of valve cuff **26** is structured to conform to irregularities around the native valve annulus, thus improving the seal between replacement valve **22** and the native valve annulus. As a result, paravalvular leakage around the replacement valve may be reduced or eliminated.

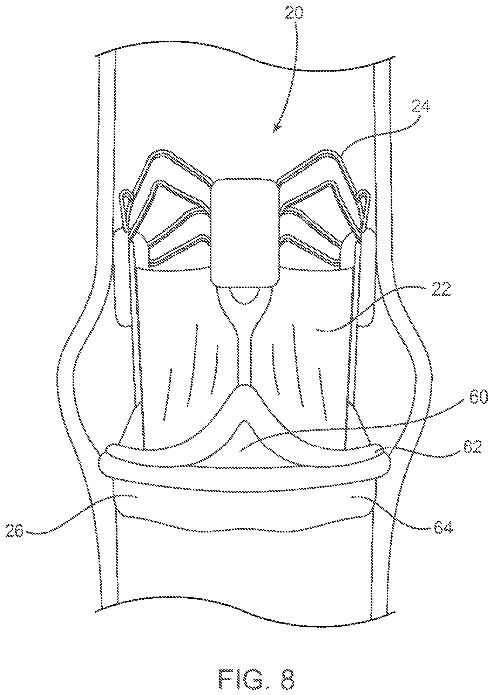

**FIG. 8** is a view of replacement valve system **20** positioned within an aortic valve, which includes native valve annulus **64.** As shown in **FIG. 8****,** valve frame **24** has expanded within the native valve annulus **64,** thereby forcing flange **62** of valve cuff **26** against native valve annulus **64** to form a tight seal between replacement valve **22** and the native valve annulus 64 so as to prevent or at least minimize paravalvular leakage and migration of replacement valve **22** from the implantation site. Thus, with flange **62** in contact with native valve annulus **64,** valve cuff **26** acts as a gasket to seal the junction between replacement valve system **20** and the native valve annulus 64.

In some embodiments, an adhesive may be applied to valve cuff **26** prior to implantation within a native valve annulus. For example, any suitable biocompatible adhesive may be applied to the outer surfaces of skirt **60** and flange **62** to help seal valve cuff **26** to the surrounding tissue of the valve annulus. While not a necessary component, biocompatible adhesives may help to provide a tighter seal in order to further reduce paravalvular leakage.

In other embodiments, the flange **62** valve cuff **26** may be constructed with a memory shaped or deformable material disposed within the flange that helps to create a tight seal with the native valve annulus. In particular, the memory shaped or deformable material may be structured to expand once valve cuff **26** is properly positioned at the implantation site. This type of valve cuff flange may be utilized regardless of whether the valve support structure is of the self-expanding or non-self-expanding type.

In some embodiments, both skirt **60** and flange **62** of valve cuff **26** can be formed from a cloth or fabric material. The fabric may comprise any suitable material including, but not limited to, woven polyester such as polyethylene terepthalate, polytetrafluoroethylene (PTFE), or other biocompatible material.

In one exemplary embodiment of assembling valve replacement system **20,** skirt **60** and flange **62** are formed as separate components that are coupled together in order to form valve cuff **26.** In particular, skirt **60** may initially be positioned around and coupled to valve support frame **24** in any suitable manner, such as by suturing. For example, each skirt attachment portion **63** may be wrapped around a corresponding support post **42** of valve frame **24.** Skirt attachment portions **63** may then, for example, be sutured to triangular shaped attachment sites **52** near the proximal ends **46** of each of the support posts **42.** Then, flange **62** may be positioned at the desired position around skirt **60** and coupled to the skirt by any suitable means, such as by suturing. Next, replacement valve **22** may be positioned within the inner lumen of frame **24,** inserting commissural tab portions **35** of replacement valve **22** through corresponding axially extending slots **44** in support posts **42.** Skirt **60** of valve **cuff 26,** which is positioned circumferentially around inflow rim **41** of frame **24,** may then be wrapped around the proximal inflow end **31** of replacement valve **22** and attached to the valve with, for example, sutures. Once attached, skirt **60** and flange **62** are structured to create tight, gasket-like sealing surfaces between replacement valve **22** and the native valve annulus. The foregoing represents only one exemplary embodiment of a method of assembling a valve replacement system in accordance with the present disclosure. Thus, modifications may be made to the number and order of steps as will be appreciate by one skilled in the art.

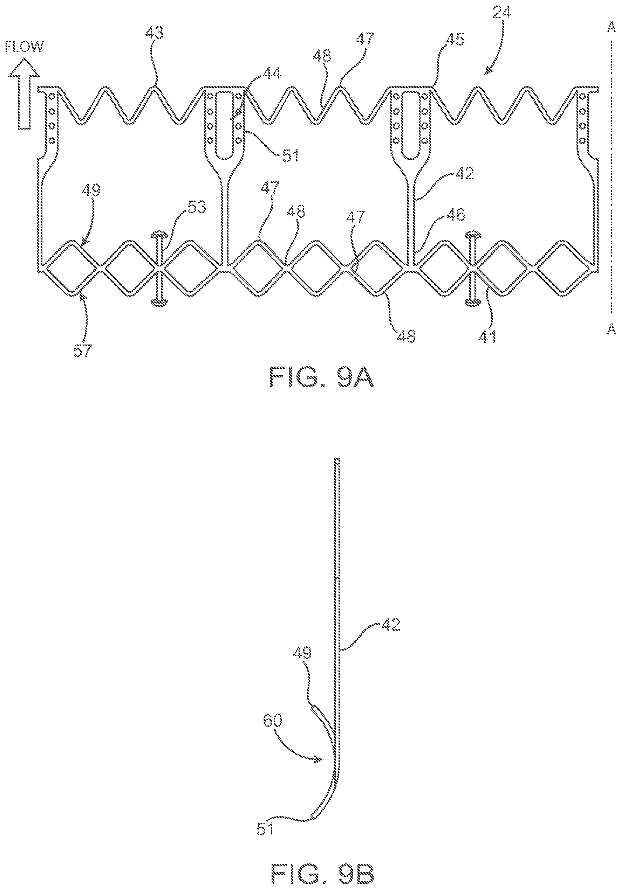

Referring now to **FIG. 9A****,** a frame **24** of a prosthetic valve may include a concave landing zone, e.g., as described in U.S. Patent Application Publication No. 2010/0100176, entitled ANCHORING STRUCTURE WITH CONCAVE LANDING ZONE. The frame **24** in **FIG. 9A** is illustrated as cut along line A-A and laid flat. The frame **24** in **FIG. 9A** represents one exemplary embodiment of a typical anchoring or support structure useable with valve replacement system **20** described herein. In general, frame **24** is designed as a collapsible and expandable anchoring structure adapted to support a valve distally along commissural region and proximally along the proximal inflow end. As shown in **FIG. 9A****,** valve has been detached from support frame **24** so as to focus on the structure and features of the support structure.

Frame **24** has a generally tubular configuration within which a replacement valve may be secured, and includes inflow rim **41,** support posts **42** and outflow rim **43.** A replacement valve may be secured at the proximal inflow end **31** by attachment to inflow rim **41** of support frame **24** and at the distal outflow end **32** via commissural tabs **35** that are threaded through axially extending slots **44,** which are formed in support posts **42** that extend longitudinally from inflow rim **41** to outflow rim **43** of valve support structure **24.** Thus, distal ends **45** of support posts **42** contact outflow rim **43** of valve support structure **24,** whereas proximal ends **46** of support posts **42** contact inflow rim **41** of frame **24.**

As shown in **FIG. 9A** outflow rim **43of** support frame **24** is depicted as comprising a single wire ring or rail that extends between support posts **42** generally at or above the axially extending slots **44** that reside therein. The outflow rim **43** is configured in an undulating or sinusoidal wave pattern forming peaks **47**and troughs **48.** However, the number of rings is not important, and numerous other configurations are contemplated and may be utilized such as single, double and triple configurations of varying patterns. Inflow rim **41** is depicted as comprising a double wire ring or rail that includes a distal inflow wire ring **49** and a proximal inflow wire ring **51.** Distal inflow wire ring **49** and proximal inflow wire ring **51** are configured in an undulating or sinusoidal wave pattern forming peaks **47** and troughs **48.** As can be seen, the double wire rail is configured so that a peak of proximal inflow wire ring **51** connects with a trough of distal inflow wire ring 51 thus forming a diamond pattern although any number of desired shapes may be achieved such as pentagonal, hexagonal, rectangular, etc., all of which are within the scope of the disclosure presented herein.

The inflow rim **41** optionally includes finger-like elements **53** positioned at which distal and proximal inflow wire rings **49, 51** connect and extend in an axial direction therefrom. Finger-like elements **53** are designed to lend additional support to fabric that may cover inflow rim 41 to anchor the fabric and permit tissue ingrowth.

In the embodiment of support frame **24** illustrated in **FIG. 9A****,** outflow rim **43** is formed with a single ring, while inflow rim 41 is formed with a double ring that extends between support posts **42.** However, the number of rings may vary, and numerous other configurations are contemplated. For example, FIG. 6A illustrates a triple ring construction for the inflow rim while FIG. 8 illustrates a single ring construction for the inflow rim.

Both inflow rim **41** and outflow rim **43** of frame **24** may be formed with an undulating or sinusoidal wave-like configurations. In various embodiments of valve support structures, inflow rim **41** may have a shorter or longer wavelength (i.e., circumferential dimension from peak to peak) or a lesser or greater wave height (i.e., axial dimension from peak to peak) than outflow rim **43.** The wavelengths and wave heights of inflow rim **41** and outflow rim **43** may be selected to ensure uniform compression and expansion of support frame **24** without substantial distortion. The wavelength of inflow rim **41** may be further selected to support the geometry of the inflow end of the valve attached thereto, such as the scalloped inflow end **31** of replacement valve **22** shown in **FIG. 9****.** Notably, as shown in **FIG. 9A****,** the undulating or sinusoidal wave pattern that forms inflow rim **41** of frame **24** may be configured such that proximal ends **46** of vertical support posts **42** are connected to troughs **48** of inflow rim **41.** Similarly, the undulating or sinusoidal wave-like pattern that forms outflow rim **43** of support structure **24** may be configured such that distal ends **45** of support posts **42** are connected at a peak **47** of outflow rim **43.** This arrangement allows the distal inflow wire ring and proximal inflow wire ring to move together when the valve is in its radially compressed state prior to delivery thus preventing possible damage to the bioprosthetic heart valve.

In the embodiment depicted in **FIG. 9A** the distal ends **45** of support posts **42** are configured generally in the shape of a paddle with axial slot **44** extending internally within blade **51** of the paddle. Blade **51** of the paddle is oriented toward outflow rim **43** of support structure **24** and connects to outflow rim **43** at a peak of the undulating sinusoidal wave-like pattern of outflow rim **43.** Support posts **42** stabilize a valve in general, and in particular the prevention of longitudinal extension at points of valve attachment to preclude valve stretching or distortion upon compression of replacement valve system. Blades **51** of the paddle-shaped support posts **42** are also designed to accommodate commissural tabs of a valve.

The number of support posts **42,** if present, generally ranges from two to four, depending on the number of commissural posts present in the valve sinus. Thus, in some embodiments, valve support structure **24** comprises three support posts for a tri-leaflet replacement valve with a native valve that features three natural commissures. Support posts **42,** if present, of frame **24** may be structured to generally coincide with the natural commissures of a native valve.

Turning now to **FIG. 9B** a cross-sectional view of the inflow rim **41** is depicted which illustrates the concave landing zone **60.** As can be seen, peaks **47** of the distal inflow ring **49** and troughs **48** of the proximal inflow ring **51** flare outwardly so that inflow rim **41** forms a C-shape in cross section upon deployment. This cross-sectional area **61** of the inflow rim **41,** or in other words the concave portion of the frame, directly corresponds to the native annulus. The frame of the inflow rim engages the native annulus, with the flared rails **47, 48** lying above and below the annulus. Upon deployment, the radial force exerted by the self-expanding frame holds the valve in position.

The concave landing zone **61** substantially prevents paravalvular leakage. Paravalvular leakage may be reduced by ensuring the inflow rim **41** is substantially secured proximally and distally of the annulus, hence forming a tight seal. Concave landing zone **60** allows the surgeon to easily place the bioprosthetic heart valve in the annulus thus minimizing patient time spent in surgery.

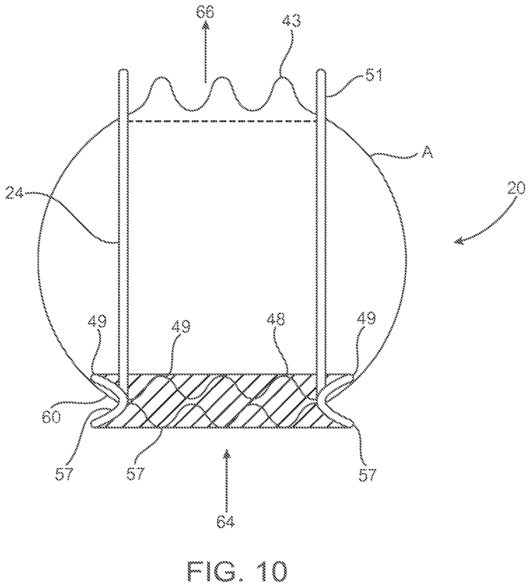

**FIG. 10** is a view of replacement valve system **20** positioned within an aorta **A,** which includes inflow annulus **64** and outflow annulus **66.** As shown in **FIG. 10****,** the tubular anchoring structure **24** of **FIG. 9A** has expanded within the sinus cavities of aorta **A**, thereby forcing inflow rim **41** against inflow annulus **64** of aorta **A** to form a tight seal between replacement valve system **20** and aorta **A.** More specifically, upon deployment inflow rim **41** assumes a substantially C-shaped in cross section concave landing zone **60** as can be seen in **FIGS. 9B** and **10****.** Distal inflow ring **49** abuts the distal side of the annulus while proximal inflow ring **51** abuts the proximal side of the native annulus. The concave landing zone **60** prevents or minimizes paravalvular leakage and migration of replacement valve system **20** from the implantation site. Thus, with inflow ring **41** in contact with inflow annulus **64,** the concave landing zone **60** acts as a gasket to seal the junction between replacement valve system **20** and aorta **A.** Typically, inflow ring **41** is covered with fabric to stimulate tissue ingrowth over time and secure the replacement heart valve in position. The fabric may comprise any suitable material including, but not limited to, woven polyester, polyester velour, polyethylene terepthalate, polytetrafluoroethylene (PTFE), or other biocompatible material. The valve assembly may be compressed in ice, loaded into a delivery system, and deployed into the aortic valve position. The self-expanding characteristic of the anchoring structure provides the radial strength required to hold the valve in position after implant

Although the above disclosure focused on a tri-leaflet replacement valve system **20**, valve cuffs in accordance with the present disclosure may be used in conjunction with any type of replacement valve of generally similar structure, including but not limited to the heart valves disclosed in U.S. application Ser. No. 10/680,071 published as US2005075731 A1 U.S. application Ser. No. 11/471092 published as US2006287718 A1, and U.S. application Ser. No. 11/489663 published as US2006259137 A1. Therefore, the valve cuff concepts disclosed herein may be applied to valve cuffs structured to function with many other types of replacement valves having any number of leaflets.

Furthermore, although the above disclosure **focuses on** frame **24** having an inflow rim **41**, an outflow rim **43,** and three support posts **42,** this particular valve support structure was described merely for purposes of example and not limitation**.** Thus, valve cuffs in accordance with the present disclosure may be used in conjunction with any generally tubular, stent-like valve support structure, as will be appreciated by one skilled in the art.

Additional designs of prosthetic heart valves for which the markings described below may be beneficial include those designs disclosed in U.S. Provisional Patent Application No. 61/819,486 filed on May 3, 2013, and those disclosed in U.S. Patent Application No. 14/268,494, entitled PROSTHETIC VALVES AND ASSOCIATED APPARATUSES. SYSTEMS AND METHODS, having attorney docket number C00005661.USU3, filed on the same day as the present application.

In embodiments, replacement valve systems described herein are sutureless valve systems. Of course, sutures may be used with such systems. Advantages to sutureless replacement valve systems include shorter implant procedure times and less invasive implantation. Some disadvantages or perceived disadvantages with current sutureless valve systems include potential increased risk of paravalvular leakage (PVL) and potential lack of durability. The designs presented herein preferably address one or more of the disadvantages or perceived disadvantages of current sutureless valve designs.

Traditionally, surgical heart valves have been implanted with a multitude of sutures, so placing the valve at the correct depth was readily accomplished by tactile means. For sutureless valves, such tactile feedback does not exist. Accordingly, alternatives for ensuring proper depth of an implant of sutureless valves would be desirable.

Additionally, ensuring proper orientation of a valve apparatus is fairly routine heart valves anchored via sutures. However, with heart valves that are expandable and initially implanted in a collapsed configuration, ensuring proper orientation can be more challenging.

In embodiments described herein, a replacement valve system **20** may include one or more markings to provide visible feedback to an implanter that the replacement valve system **20** is implanted in a desired location, position, or orientation, such as at a proper depth. According to the invention, the marking comprises a marking around a circumference of the valve apparatus **20.** In some embodiments, the marking comprises a circumferential marking at a location of the valve apparatus to be aligned with one or more structures of a patient's anatomy such as one or more structures related to a patient's valve that requires replacement..

In embodiments, described herein, a heart valve apparatus includes a marking to provide visible feedback to an implanter that correct rotational orientation of the valve apparatus is achieved. In embodiments, the marking comprises a marking along at least a portion of the length of the valve apparatus. In embodiments, the marking is configured to be aligned with a commissure of the patient's valve.

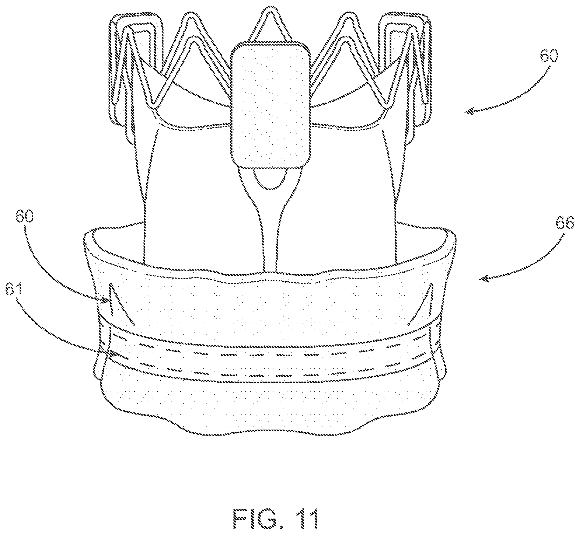

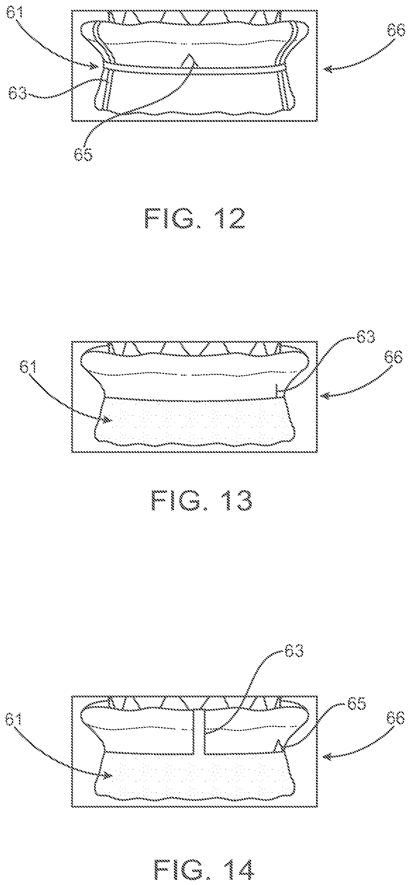

With the above general description in mind, reference is now made to **FIGS. 11-15****,** in which schematic drawings of a valve apparatus **60** or close up of a skirt **66** of a valve apparatus are shown. In the depicted embodiments, markings **61, 63** and **65** are presented on, or are visible through, skirt **66.** Markings **61** are circumferential and may be used to provide visual feedback regarding proper depth of implantation. In the embodiments depicted in **FIGS. 11-12****,** markings **61** represent the position of proper alignment with the annulus of patient's valve. That is, if marking **61** is aligned with the patient's annulus, the valve apparatus is positioned at the proper depth. In the embodiments depicted in **FIGS. 13-14****,** the top edge of markings **61** represent the position of proper alignment with the patient's annulus. As a valve apparatus as depicted in **FIG. 13** or **FIG. 14** is being implanted, marking **61** should be visible until the valve apparatus is properly positioned. That is, when the upper edge of marking **61** is aligned with the patient's annulus (and the remainder of the marking is below the annulus), the marking **61** should no longer be visible to an implanter.

Circumferential marking **61** may be in the form of a circumferential line, which may be solid or dashed. Circumferential marking **61** may be in the form of a transition edge, where the skirt is one color or pattern below the edge and another color or pattern above the edge. Circumferential marking **61** may be formed from die, ink, thread, band or ribbon, or the like. Circumferential marking **61** may be formed from a transition between two types of materials. For example, the "bottom half' may be formed from one material and the "top half" may be formed from another material. Any suitable materials may be used to create a demarcation or delineation line, such as fabric, polymer, tissue or the like.

In embodiments, a ribbon may be stitched around the circumference of a skirt to form a circumferential marking. The ribbon or stitching may be colored. The ribbon may be placed on the skirt before or after the skirt is placed on the valve apparatus. The ribbon may be formed of any suitable material, such as cottony Dacron, cottony II or the like.

In **FIGS. 11-14****,** markings **63** are configured to be aligned with a commissure of the patient's valve. When marking **63** is aligned with a commissure, the valve apparatus may be expanded (or allowed to expand) so that the valve apparatus will be in proper rotational orientation relative to the patient's valve. A valve apparatus may include more than one commissural-alignment marking **63** (see, e.g., **FIG. 12****).** A commissural-alignment marking **63** may extend the length of the skirt **66** (see. e.g., **FIG. 12** and **FIG. 14**) of along a portion of the length of the skirt **66** (see, e.g., **FIG. 13****).**

Commissural-alignment markings **63** may be formed in any suitable manner: e.g., die, ink, thread, band or ribbon, or the like. Vertical markings **63** can be of any suitable width, can be solid or dashed, or the like.

Veritical markings **63** may also serve the purpose of identifying where the commissures of the bioprosthesis valve apparatus are located. In this manner the user can check that the bioprosthesis valve apparatus commissures will not block coronary flow.

Also shown in **FIGS. 11****,** **12 & 14** are suture markings **65.** Suture markings **65** may be used to indicate the desired location of any guiding sutures an implanter may wish to place to lower the valve to a particular depth, similar to how a stented valve is implanted but with fewer stitches. Suture markings **63** may be formed in any suitable manner: e.g., die, ink, thread, band or ribbon, or the like.

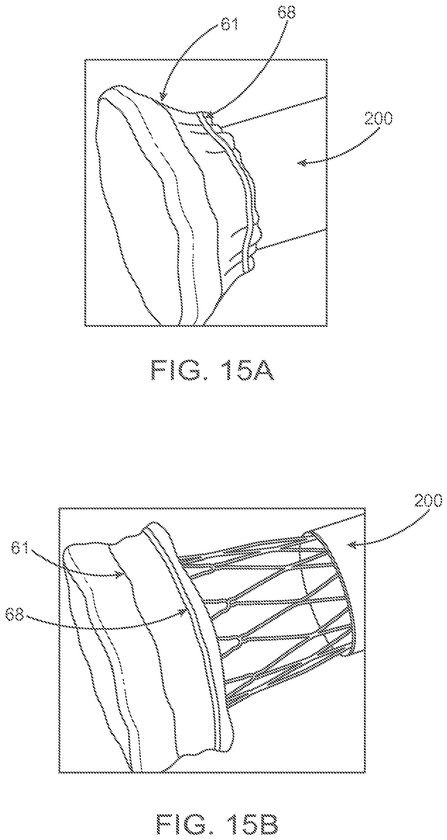

Referring now to **FIGS. 15A-B,** a prosthetic valve having first **61** and second **68** circumferential markings is shown that falls within the scope of the claims. The first marking **61** is an annulus alignment marking (e.g., as discussed above with regard to **FIGS. 11-14****).** The second marking **68** is a marking to indicate a position at which a valve retention sheath **200** may be withdrawn over the valve device during an intermediate stage of implanting the valve device. The inflow portion of the valve device may be positioned inferior to the annulus of the native valve with the sheath **200** covering most. if not all, of the valve device. Once the inflow portion of the valve device is positioned inferior to the native valve annulus, the sheath **200** may be retracted until the second circumferential marking **68** is visible, which in the depicted embodiment allows the lower inflow portion of the valve device to expand (and engage a portion of the vessel inferior to the annulus of the native valve). As the first marking **61** is also visible with the sheath retracted just beyond the second circumferential marking **68**, the implanter can adjust the depth of the valve device in until the first circumferential marking **61** is aligned with the native valve annulus. When the first circumferential marking **61** is aligned with the native valve annulus, the sheath **200** may be further retracted over the valve device to allow the upper inflow portion of the valve frame to expand; e.g., as shown in **FIG. 15B****.** In the depicted embodiment, the inflow portion of the frame of the valve device is concave in its expanded configuration. When expanded the lower inflow and upper inflow portions of the frame are configured to engage the patient's anatomy inferior to and superior to, respectively, the annulus of the native valve and to cooperate to prevent lateral movement of the valve device when implanted.

The valve retention sheath **200** shown in **FIGS. 15A** and **15B** may be a part of a valve delivery system as generally known to those of skill in the art.

The markings depicted in **FIGS. 11-15** are on, or visible through the skirt. Further disclosed but not claimed is that markings may be placed at any other suitable location of the valve apparatus In certain embodiments, one or more markings of the present invention may be configured to be visualized using one or more medical imaging techniques, e.g., one or more markings may comprise one or more radiopaque materials.

In one or more embodiments, valve prosthesis may comprise a balloon-expandable, mechanically-expandable, or a self-expandable frame that may be collapsed during delivery and expanded upon deployment within a native valve. The frame may be self-expanding via removal of external compressive forces or expanded using an outward radial force (e.g., balloon or mechanical expansion).In one or more embodiments, valve prosthesis or one or more of its components or portions may be positioned in, positioned through, or positioned adjacent to, for example, a natural valve, a native valve, a synthetic valve, a replacement valve, a tissue valve, a mechanical valve, a mitral valve, an aortic valve, a pulmonary valve, a tricuspid valve, a valve component, a valve annulus, a valve leaflet, chordea, or a valve commissure.

In one or more embodiments, valve prosthesis may be implanted into an annulus of a native cardiac valve via a suitable delivery route or procedure. For example, the valve prosthesis may be delivered through an artery or vein, a femoral artery, a femoral vein, a jugular vein, a subclavian artery, an axillary artery, an aorta, an atrium, or a ventricle. The valve prosthesis may be delivered via a transfemoral, transapical, transseptal, transatrial, transventrical, transaortic, transcatheter, surgical, beating heart, stopped heart, pump-assisted, or a cardiopulmonary bypass procedure.

In one or more embodiments, valve prosthesis or one or more of its components or portions may be delivered, for example, through a thoracotomy, a sternotomy, percutaneously, transvenously, arthroscopically, endoscopically, for example, through a percutaneous port, a stab wound or puncture, through a small incision, for example, in the chest, groin, abdomen, neck, leg, arm, or in combinations thereof.

In certain embodiments, the valve prosthesis is configured for replacing an aortic valve. Alternatively, other shapes are also envisioned to adapt to the specific anatomy of the valve to be replaced (e.g., stented prosthetic heart valves in accordance with the present disclosure can be shaped or sized for replacing a native aortic, mitral, pulmonic or tricuspid valve).In one or more embodiments, valve prosthesis or one or more of its components or portions may comprise, be covered with, be coated with, or be attached or coupled to one or more biocompatible materials or biomaterials, for example, titanium, titanium alloys, Nitinol, TiNi alloys, shape memory alloys, super elastic alloys, aluminum oxide, platinum, platinum alloys, stainless steels, stainless steel alloys, MP35N, elgiloy, haynes 25, stellite, pyrolytic carbon, silver carbon, glassy carbon, polymers or plastics such as polyamides, polycarbonates, polyethers, polyesters, polyolefins including polyethylenes or polypropylenes, polystyrenes, polyurethanes, polyvinylchlorides, polyvinylpyrrolidones, silicone elastomers, fluoropolymers, polyacrylates, polyisoprenes, polytetrafluoroethylenes, polyethylene terephthalates, fabrics such as woven fabrics, nonwoven fabrics, porous fabrics, semiporous fabrics, nonporous fabrics, Dacron fabrics, polytetrafluoroethylene (PTFE) fabrics, polyethylene terephthalate (PET) fabrics, materials that promote tissue ingrowth, rubber, minerals, ceramics, hydroxapatite, epoxies, human or animal protein or tissue such as collagen, laminin, elastin or fibrin, organic materials such as cellulose, or compressed carbon, or other materials such as glass, and the like. Materials that are not considered biocompatible may be modified to become biocompatible by a number of methods well known in the art. For example, coating a material with a biocompatible coating may enhance the biocompatibility of that material. Biocompatible materials or biomaterials are usually designed and constructed to be placed in or onto tissue of a patient's body or to contact fluid of a patient's body. Ideally, a biocompatible material or biomaterial will not induce undesirable reactions in the body such as blood clotting, tumor formation, allergic reaction, foreign body reaction (rejection) or inflammatory reaction; will have the physical properties such as strength, elasticity, permeability, and flexibility required to function for the intended purpose; may be purified, fabricated and sterilized easily; will substantially maintain its physical properties and function during the time that it remains in contact with tissues or fluids of the body.

There are several contemplated methods for implanting the valve replacement systems described above. In a first method, the patient is placed on cardiopulmonary bypass. A small incision is made on the upper sternum to access the ascending aorta. The aorta is clamped and opened to expose the diseased aortic valve, which is excised. The replacement valve system is then inserted within the aorta under direct vision. The valve cuff coupled to the replacement valve thereafter assists in both fixing the valve to the aortic valve annulus and preventing or reducing paravalvular leakage by forming a tight seal with aortic valve annulus.

In a second method, a self-expanding valve is collapsed and delivered in a collapsed state. Once the valve is properly positioned within the native valve, the valve is deployed, thereby allowing the valve to expand into position with the valve cuff pushing against the valve annulus to form a tight seal. In such an embodiment, the self-expanding valve includes a self-expanding frame that is structured to provide the radial force necessary to push the cuff against the native valve annulus.

In a third method, a non-self-expanding valve is "rolled" up and delivered to the native valve. Once property positioned within the native valve, the valve cuff is pushed against the native valve annulus by "unrolling" the replacement valve.

One skilled in the art will appreciate that although only three replacement valve implantation methods are described herein, numerous other methods are possible and within the intended scope of the present disclosure. Thus, the three exemplary implantation methods are provided for purposes of example and not limitation.

Implant methods, valve delivery systems and associated devices that may be employed with the replacement valve systems described herein are disclosed in U.S. Patent Application No. 14J268375, entitled VALVE DELIVERY TOOL, having attorney docket no. C00001363.USU2, filed on the same day as the present application.

### Definitions

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, the term "or" is generally employed in its sense including "and/or" unless the content clearly dictates otherwise. The term "and/or" means one or all of the listed elements of a combination of any two or more of the listed elements.

As used herein, "have", "having", "include", "including", "comprise", "comprising" or the like are used in their open ended sense, and generally mean "including, but not limited to". It will be understood that "consisting essentially of", "consisting of", and the like are subsumed in "comprising" and the like. As used herein, "consisting essentially of," as it relates to **a** composition, article, system, method or the like. means that the components of the composition, article, system, method or the like are limited to the enumerated components and any other components that do not materially affect the basic and novel characteristic(s) of the composition, article, system, method or the like.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

Also herein, the recitations of numerical ranges by endpoints include all numbers subsumed within that range (e.g., 1 to 5 includes 1, 1.5, 2, 2.75, 3, 3.80, 4, 5, etc. or 10 or less includes 10, 9.4, 7.6, 5, 4.3, 2.9, 1.62, 0.3, etc.). Where a range of values is "up to" a particular value, that value is included within the range.

As used herein, the term "about" encompasses the range of experimental error that occurs in any measurement.

As used herein, "exemplary" means serving as an example and does not necessarily imply that the example is preferable or the best of its kind.

In the detailed description above several specific embodiments of compounds, compositions, articles, systems and methods are disclosed. The detailed description, therefore, is not to be taken in a limiting sense.

Thus, embodiments of MEDICAL DEVICES FOR IMPLANTING IN A VALVE AND ASSOCIATED METHODS are disclosed. One skilled in the art will appreciate that the heart valves and associated apparatuses, systems and methods described herein can be practiced with embodiments other than those disclosed. The disclosed embodiments are presented for purposes of illustration and not limitation.

## Claims

1. A device (20) configured to be implanted in a valve of a subject, the valve comprising an annulus (64), and the device comprising:
a frame (24) having an annular portion configured to be aligned with the annulus (64) of the valve;
a visible circumferential marking (61), configured to be aligned with the annulus, and positioned around the annular portion of the frame (24);
a skirt (60, 66) disposed over at least a portion of the frame (24), wherein the visible circumferential marking (61) is presented on or visible through the skirt (60, 66); and
**characterized in that** the device also comprises
a second visible circumferential marking (61) positioned around the frame (24) at a location that, when implanted, is superior to the first visible circumferential marking (61), wherein the second circumferential marking (61) indicates a position to which a sheath of a delivery system is to be withdrawn to allow the device to partially expand during a part of an implant procedure in which the device (20) is being positioned within the valve.

2. A device (20) according to claim 1, wherein the frame (24) is expandable from a collapsed configuration to an expanded configuration, and wherein, in the expanded configuration, the annular portion of the frame (24) is configured to engage the annulus (64) of the valve.

3. A device (20) according to claim 2, wherein in the frame (24) is configured to be at least partially collapsed from the expanded configuration to an at least partially collapsed configuration such that the frame (24) can be repositioned during an implant procedure if the visible circumferential marking (61) is not aligned with the annulus (64) of the valve.

4. A device (20) according to claim 1, wherein the frame (24) is expandable from a collapsed configuration to a partially expanded configuration, and wherein, in the partially expanded configuration, the device (20) is configured such that circumferential marking (61) is visible when aligned with the annulus (64) of the valve.

5. A device (20) according to claim 1, wherein the visible circumferential marking (61) is formed from one or more of a die, an ink, a thread, and a band.

6. A device (20) according to claim 1, wherein the visible circumferential marking (61) comprises a transition from a first color, material or pattern to a second color, material or pattern; wherein optionally the visible circumferential marking (61) comprises an edge of a ribbon.

7. A device (20) according to claim 1, wherein the frame (24) has a longitudinal portion configured to be aligned with a commissure of the valve, and wherein the device (20) further comprises one or more visible commissural alignment markings positioned to indicate at least a portion of the longitudinal portion of the frame (24).

8. A device (20) according to claim 7, wherein the one or more visible commissural alignment markings are disposed on the skirt (60, 66).

9. A device (20) according to claim 7, wherein the frame (24) has a plurality of longitudinal portions, each configured to be aligned with one of a plurality of commissures of the valve sinus, and wherein the device further comprises a plurality of one or more visible commissural alignment markings, each of the plurality of one or more markings positioned to indicate at least a portion of one of the plurality of the longitudinal portions of the frame (24).

10. A device (20) according to claim 1, wherein the frame (24) comprises a flange positioned superior to the annular portion when implanted, wherein the flange is compressible and expandable.

11. A device according to claim 10, wherein the flange is a part of a concave-shaped portion configured to anchor the device around the annulus (64).

12. A device (20) according to claim 1, wherein the device is a prosthetic valve, and wherein the device further comprises a valve leaflet disposed within the frame (24).

13. A device (20) according to claim 12, wherein the device is a prosthetic heart valve.

14. A device (20) according to claim 1, wherein the device is a sutureless prosthetic heart valve.

## Patentansprüche

1. Vorrichtung (20), die konfiguriert ist, um in eine Klappe eines Subjekts implantiert zu werden, die Klappe umfassend einen Ring (64) und die Vorrichtung umfassend:
einen Rahmen (24), der einen ringförmigen Abschnitt aufweist, der konfiguriert ist, um mit dem Ring (64) der Klappe ausgerichtet zu werden;
eine sichtbare Umfangsmarkierung (61), die konfiguriert ist, um mit dem Ring ausgerichtet zu werden, und um den ringförmigen Abschnitt des Rahmens (24) positioniert ist;
einen Mantel (60, 66), der über mindestens einem Abschnitt des Rahmens (24) angeordnet ist, wobei die sichtbare Umfangsmarkierung (61) an dem Mantel (60, 66) gezeigt oder durch diesen sichtbar ist; und
**dadurch gekennzeichnet, dass** die Vorrichtung auch eine zweite sichtbare Umfangsmarkierung (61) umfasst, die um den Rahmen (24) an einem Ort positioniert ist, der, wenn implantiert, höher als die erste sichtbare Umfangsmarkierung (61) ist, wobei die zweite Umfangsmarkierung (61) eine Position angibt, zu der eine Hülle eines Zuführsystems zurückgezogen werden soll, um es der Vorrichtung zu ermöglichen, sich während eines Teils eines Implantatvorgangs, in dem die Vorrichtung (20) innerhalb der Klappe positioniert wird, teilweise zu erweitern.

2. Vorrichtung (20) nach Anspruch 1, wobei der Rahmen (24) von einer zusammengeschobenen Konfiguration in eine erweiterte Konfiguration erweiterbar ist, und wobei in der erweiterten Konfiguration der ringförmige Abschnitt des Rahmens (24) konfiguriert ist, um in den Ring (64) der Klappe einzugreifen.

3. Vorrichtung (20) nach Anspruch 2, wobei in dem Rahmen (24) konfiguriert ist, um mindestens teilweise von der erweiterten Konfiguration in eine mindestens teilweise zusammengeschobene Konfiguration derart zusammengeschoben zu sein, dass der Rahmen (24) während eines Implantatvorgangs neu positioniert werden kann, falls die sichtbare Umfangsmarkierung (61) nicht mit dem Ring (64) der Klappe ausgerichtet ist.

4. Vorrichtung (20) nach Anspruch 1, wobei der Rahmen (24) von einer zusammengeschobenen Konfiguration in eine teilweise erweiterte Konfiguration erweiterbar ist, und wobei in der teilweise erweiterten Konfiguration die Vorrichtung (20) derart konfiguriert ist, dass die Umfangsmarkierung (61) sichtbar ist, wenn sie mit dem Ring (64) der Klappe ausgerichtet ist.

5. Vorrichtung (20) nach Anspruch 1, wobei die sichtbare Umfangsmarkierung (61) aus einem oder mehreren von einem Werkzeug, einer Tinte, einem Faden und einem Streifen ausgebildet ist.

6. Vorrichtung (20) nach Anspruch 1, wobei die sichtbare Umfangsmarkierung (61) einen Übergang von einer ersten Farbe, einem ersten Material oder Muster zu einer zweiten Farbe, einem zweiten Material oder Muster umfasst; wobei optional die sichtbare Umfangsmarkierung (61) eine Kante eines Bandes umfasst.

7. Vorrichtung (20) nach Anspruch 1, wobei der Rahmen (24) einen Längsabschnitt aufweist, der konfiguriert ist, um mit einer Kommissur der Klappe ausgerichtet zu werden, und wobei die Vorrichtung (20) ferner eine oder mehrere sichtbare Kommissurenausrichtungsmarkierungen umfasst, die positioniert sind, um mindestens einen Abschnitt des Längsabschnitts des Rahmens (24) anzugeben.

8. eine Vorrichtung (20) nach Anspruch 7,
wobei die eine oder die mehreren sichtbaren Kommissurenmarkierungen auf dem Mantel (60, 66) angeordnet sind.

9. Vorrichtung (20) nach Anspruch 7, wobei der Rahmen (24) eine Vielzahl von Längsabschnitten aufweist, die jeweils konfiguriert sind, um mit einer von einer Vielzahl von Kommissuren des Klappensinus ausgerichtet zu werden, und wobei die Vorrichtung ferner eine Vielzahl von einer oder mehreren sichtbaren Kommissurenausrichtungsmarkierungen umfasst, wobei jede der Vielzahl von einer oder mehreren Markierungen positioniert ist, um mindestens einen Abschnitt eines der Vielzahl der Längsabschnitte des Rahmens (24) anzugeben.

10. Vorrichtung (20) nach Anspruch 1, wobei der Rahmen (24) einen Flansch umfasst, der höher als der ringförmige Abschnitt positioniert ist, wenn implantiert, wobei der Flansch komprimierbar und erweiterbar ist.

11. Vorrichtung nach Anspruch 10, wobei der Flansch ein Teil eines konkav geformten Abschnitts ist, der konfiguriert ist, um die Vorrichtung um den Ring (64) zu verankern.

12. Vorrichtung (20) nach Anspruch 1, wobei die Vorrichtung eine Klappenprothese ist und wobei die Vorrichtung ferner ein Klappensegel umfasst, das innerhalb des Rahmens (24) angeordnet ist.

13. Vorrichtung (20) nach Anspruch 12, wobei die Vorrichtung eine Herzklappenprothese ist.

14. Vorrichtung (20) nach Anspruch 1, wobei die Vorrichtung eine nahtlose Herzklappenprothese ist.

## Revendications

1. Dispositif (20) configuré pour être implanté dans une valvule d'un sujet, la valvule comprenant un anneau (64), et le dispositif comprenant :
une armature (24) ayant une partie annulaire configurée pour être alignée sur l'anneau (64) de la valvule ;
un marquage circonférentiel visible (61), configuré pour être aligné sur l'anneau, et positionné autour de la partie annulaire de l'armature (24) ;
une jupe (60, 66) disposée sur au moins une partie de l'armature (24), dans lequel le marquage circonférentiel visible (61) est présenté sur la jupe (60, 66) ou visible à travers celle-ci ; et
**caractérisé en ce que** le dispositif comprend également un second marquage circonférentiel visible (61) positionné autour de l'armature (24) au niveau d'un emplacement qui, lorsqu'il est implanté, est au-dessus du premier marquage circonférentiel visible (61), dans lequel le second marquage circonférentiel (61) indique une position à laquelle une gaine d'un système de pose doit être retirée pour permettre au dispositif de se détendre partiellement pendant une partie d'une intervention d'implantation dans laquelle le dispositif (20) est en cours de positionnement à l'intérieur de la valvule.

2. Dispositif (20) selon la revendication 1, dans lequel l'armature (24) est extensible d'une configuration repliée à une configuration détendue, et dans lequel, dans la configuration détendue, la partie annulaire de l'armature (24) est configurée pour venir en prise avec l'anneau (64) de la valvule.

3. Dispositif (20) selon la revendication 2, dans lequel dans l'armature (24) est configurée pour être au moins partiellement repliée de la configuration étendue à une configuration au moins partiellement repliée de telle sorte que l'armature (24) peut être repositionnée pendant une intervention d'implantation si le marquage circonférentiel visible (61) n'est pas aligné sur l'anneau (64) de la valvule.

4. Dispositif (20) selon la revendication 1, dans lequel l'armature (24) est extensible d'une configuration repliée à une configuration partiellement étendue, et dans lequel, dans la configuration partiellement étendue, le dispositif (20) est configuré de telle sorte que le marquage circonférentiel (61) est visible lorsqu'il est aligné sur l'anneau (64) de la valvule.

5. Dispositif (20) selon la revendication 1, dans lequel le marquage circonférentiel visible (61) est formé à partir d'un ou plusieurs parmi une filière, une encre, un fil, et une bande.

6. Dispositif (20) selon la revendication 1, dans lequel le marquage circonférentiel visible (61) comprend une transition d'une première couleur, d'un premier matériau ou d'un premier motif à une seconde couleur, un second matériau ou un second motif ; dans lequel facultativement le marquage circonférentiel visible (61) comprend un bord d'un ruban.

7. Dispositif (20) selon la revendication 1, dans lequel l'armature (24) a une partie longitudinale configurée pour être alignée sur une commissure de la valvule, et dans lequel le dispositif (20) comprend en outre un ou plusieurs marquages d'alignement commissuraux visibles positionnés pour indiquer au moins une partie de la partie longitudinale de l'armature (24).

8. Dispositif (20) selon la revendication 7,
dans lequel les un ou plusieurs marquages d'alignement commissuraux visibles sont disposés sur la jupe (60, 66).

9. Dispositif (20) selon la revendication 7, dans lequel l'armature (24) a une pluralité de parties longitudinales, chacune configurée pour être alignée sur l'une d'une pluralité de commissures du sinus valvulaire, et dans lequel le dispositif comprend en outre une pluralité d'un ou plusieurs marquages d'alignement commissuraux visibles, chaque marquage de la pluralité de marquages étant positionnés pour indiquer au moins une partie d'une partie de la pluralité des parties longitudinales de l'armature (24).

10. Dispositif (20) selon la revendication 1, dans lequel l'armature (24) comprend une bride positionnée au-dessus de la partie annulaire lorsqu'elle est implantée, dans lequel la bride est compressible et extensible.

11. Dispositif selon la revendication 10, dans lequel la bride est une partie d'une partie en forme concave configurée pour ancrer le dispositif autour de l'anneau (64).

12. Dispositif (20) selon la revendication 1, dans lequel le dispositif est une valvule prothétique, et dans lequel le dispositif comprend en outre un feuillet valvulaire disposé à l'intérieur de l'armature (24).

13. Dispositif (20) selon la revendication 12, dans lequel le dispositif est une valvule cardiaque prothétique.

14. Dispositif (20) selon la revendication 1, dans lequel le dispositif est une valvule cardiaque prothétique sans suture.
